# FASCICULE DE BREVET EUROPEEN

(11) **EP 2 652 509 B1**
(45) Date de publication et mention de la délivrance du brevet: **18.11.2015**
(21) Numéro de dépôt: 11817337.6
(22) Date de dépôt: 14.12.2011
(51) Int. Cl.: C12Q 1/68, G01N 33/92, G01N 33/576

(54) **PROCÉDÉ POUR PRÉDIRE LA RÉPONSE À UN TRAITEMENT CONTRE L'HÉPATITE C**
VERFAHREN ZUR VORHERSAGE DER REAKTION AUF EINE BEHANDLUNG GEGEN HEPATITIS C
METHOD FOR PREDICTING THE RESPONSE TO A TREATMENT AGAINST HEPATITIS C

(30) Priorité: 15.12.2010 FR 1060568
(43) Date de publication de la demande: 23.10.2013
(73) Titulaire: Centre Hospitalier Universitaire de Montpellier, 34000 Montpellier (FR); Université Montpellier 1, 34967 Montpellier Cedex 2 (FR)
(72) Inventeur: LEHMANN, Sylvain, F-34170 Castelnau-le-Lez (FR); ROCHE, Stéphane, 13480 Cabries (FR); DUCOS, Jacques, F-34000 Montpellier (FR); TIERS, Laurent, 34090 Montpellier (FR)
(74) Mandataire: Nevant, Marc
(86) Numéro de dépôt international: PCT/FR2011/052989
(87) Numéro de publication internationale: WO 2012/080662

(56) Documents cités:
- EP-A1- 1 953 242
- WO-A1-2006/107078
- WO-A2-2010/076788
- FUJITA NAOKI ET AL: "Identification of Treatment Efficacy-Related Host Factors in Chronic Hepatitis C by Protein Chip Serum Analysis", MOLECULAR MEDICINE (BALTIMORE), vol. 17, no. 1-2, 5 octobre 2010 (2010-10-05), pages 70-78, XP002636430, ISSN: 1076-1551
- BISIAUX A ET AL: "Molecular Analyte Profiling of the Early Events and Tissue Conditioning Following Intravesical Bacillus Calmette-Guerin Therapy in Patients With Superficial Bladder Cancer", JOURNAL OF UROLOGY, LIPPINCOTT WILLIAMS & WILKINS, BALTIMORE, MD, US, vol. 181, no. 4, 1 avril 2009 (2009-04-01) , pages 1571-1580, XP026013926, ISSN: 0022-5347, DOI: 10.1016/J.JURO.2008.11.124 [extrait le 2009-02-23]
- SONIA MOLINA ET AL: "Identification of apolipoprotein C-III as a potential plasmatic biomarker associated with the resolution of hepatitis C virus infection", PROTEOMICS - CLINICAL APPLICATIONS, vol. 2, no. 5, 1 mai 2008 (2008-05-01), pages 751-761, XP055027363, ISSN: 1862-8346, DOI: 10.1002/prca.200800020
- ROMERO ANA I ET AL: "Interferon (IFN)-gamma-inducible protein-10: association with histological results, viral kinetics, and outcome during treatment with pegylated IFN-alpha 2a and ribavirin for chronic hepatitis C virus infection", JOURNAL OF INFECTIOUS DISEASES, UNIVERSITY OF CHICAGO PRESS, CHICAGO, IL, vol. 194, no. 7, 1 octobre 2006 (2006-10-01), pages 895-903, XP009099562, ISSN: 0022-1899, DOI: 10.1086/507307
- Christopher J Deighan ET AL: "Patients with nephrotic-range proteinuria have apolipoprotein C and E deficient VLDL 1", Kidney International, 1 January 2000 (2000-01-01), pages 1238-1246, XP055129809, Retrieved from the Internet: URL:http://www.nature.com/ki/journal/v58/n 3/pdf/4495482a.pdf [retrieved on 2014-07-17]

### SEQUENCE LISTING

<110> CENTRE HOSPITALIER UNIVERSITAIRE DE MONTPELLIER
<120> Procédé pour prédire la réponse à un traitement contre l'hépatite C
<130> 1H265100 0003 WO/MN
<150> FR1060568
   <151> 2010-12-15
<160> 5
<170> PatentIn version 3.5
<210> 1
   <211> 16
   <212> PRT
   <213> Homo sapiens
<400> 1
   Asp Ala Leu Ser Ser Val Gln Glu Ser Gln Val Ala Gln Gln Ala Arg
   1               5                   10                  15
<210> 2
   <211> 19
   <212> PRT
   <213> Homo sapiens
<400> 2
   Phe Ser Glu Phe Trp Asp Leu Asp Pro Glu Val Arg Pro Thr Ser Ala
   1               5                   10                  15
   Val Ala Ala
<210> 3
   <211> 11
   <212> PRT
   <213> Homo sapiens
<400> 3
   Gly Trp Val Thr Asp Gly Phe Ser Ser Leu Lys
   1               5                   10
<210> 4
   <211> 21
   <212> PRT
   <213> Homo sapiens
<400> 4
   Asp Lys Phe Ser Glu Phe Trp Asp Leu Asp Pro Glu Val Arg Pro Thr
   1 5 10 15
   Ser Ala Val Ala Ala
   20
<210> 5
   <211> 99
   <212> PRT
   <213> Homo sapiens
<220>
   <221> SIGNAL
   <222> (1) .. (20)
<400> 5
   Met Gln Pro Arg Val Leu Leu Val Val Ala Leu Leu Ala Leu Leu Ala
   1 5 10 15
   Ser Ala Arg Ala Ser Glu Ala Glu Asp Ala Ser Leu Leu Ser Phe Met
   20 25 30
   Gln Gly Tyr Met Lys His Ala Thr Lys Thr Ala Lys Asp Ala Leu Ser
   35 40 45
   Ser Val Gln Glu Ser Gln Val Ala Gln Gln Ala Arg Gly Trp Val Thr
   50 55 60
   Asp Gly Phe Ser Ser Leu Lys Asp Tyr Trp Ser Thr Val Lys Asp Lys
   65 70 75 80
   Phe Ser Glu Phe Trp Asp Leu Asp Pro Glu Val Arg Pro Ala Ser Ala
   85 90 95
   Val Ala Ala

## Description

### Domaine technique de l'invention

La présente invention concerne le domaine du diagnostic. Plus particulièrement, l'invention concerne une méthode pour prédire l'efficacité d'un traitement de l'hépatite C.

### Etat de la technique

Selon le Bulletin épidémiologique hebdomadaire de l'Institut de Veille Sanitaire en date du 1er Juillet 2008, le nombre annuel de décès associés au virus de l'hépatite C (VHC) en 2001 était de 3 618 (respectivement 6,1 décès pour 100 000 habitants). Le nombre annuel de décès imputables au VHC était 2 646 (4,5 pour 100 000 habitants). 95 % avaient une cirrhose et 33 % un carcinome hépatocellulaire. Cette mortalité devrait augmenter dans les années à venir sachant que l'augmentation très importante du nombre de cancer primitif du foie dans les pays occidentaux est en grande partie liée au virus de l'hépatite C.

Actuellement, trois types de thérapies sont disponibles pour soigner l'infection à VHC :
- le traitement par l'interféron, efficace chez environ 20 % des personnes infectées par le VHC ;
- l'association interféron pégylé-ribavirine, efficace chez environ 50 % des personnes infectées par le VHC ;
- l'interféron mis au point par des techniques biologiques, efficace chez environ 60 à 70 % des personnes infectées par le VHC.

Tous ces traitements, qui sont administrés par injection, ont des effets indésirables tels que la fièvre, les maux de tête, la fatigue, la nausée et les vomissements. En outre, le traitement modifie la formule sanguine.

Chez les patients atteints d'hépatite chronique C, l'association interféron pégyléribavirine entraîne une réponse virale prolongée dans 55 % des cas. L'obtention d'une réponse virologique prolongée est l'objectif principal du traitement de l'hépatite chronique C (environ 60 à 80 % des patients infectés par le VHC). Elle est associée à la disparition de l'activité histologique et éventuellement de la fibrose hépatique, ainsi qu'à une réduction du risque de carcinome hépatocellulaire.

Les patients non répondeurs (45%) peuvent être identifiés par la détection de la charge virale au mieux au bilan de la 12^{ème} semaine, voire à l'arrêt du traitement (6 ou 12 mois suivant le génotype). La charge virale présente à la 12^{ème} semaine peut conditionner l'arrêt thérapeutique.

La non réponse au traitement est un problème en particulier pour les patients infectés par le génotype 1 du virus qui ne répondent favorablement que dans 40-50% des cas par rapport aux patients infectés par les génotypes 2 et 3 qui ont 80-90% de réponse au traitement.

Une étude récente a mis en évidence des facteurs qui influencent la réponse au traitement dont l'âge, le génotype viral, l'indice de masse corporelle (Elefsiniotis *et al.*, 2008). Mais ces éléments ne sont pas assez performants pour dicter un choix thérapeutique.

En étudiant *in vitro* les mécanismes de résistance au traitement du VHC, il apparaît que différentes protéines virales (E2, NS3/4A et NS5A) puissent y jouer un rôle (Hofmann *et al.*, 2005). Dans la majorité des études cliniques il n'a pourtant pas été possible de lier les variations de séquence de ces protéines à la réponse au traitement (Wohnsland *et al.*, 2007). En parallèle des approches génomiques globales (puce, SNP - de l'anglais 'single nucleotide polymorphism') ont été menées pour trouver des marqueurs prédictifs de réponse au traitement. Certains polymorphismes du promoteur de l'IL10 (Morgan *et al.*, 2008) ont été suggérés mais c'est en fait un marqueur nommé IL28B (Ge *et al.*, 2009) qui semble montrer un intérêt actuellement en clinique. Des marqueurs de la réponse à un traitement à base d'interféron chez des patients atteints d'hépatite C chronique ont été identifiés par spectrométrie SELDI-TOF (Fujita et al., 2011). L'apolipoproétine C-III a été identifiée comme biomarqueur plasmatique potentiel associé à la résolution de l'infection par le VHC (Molina et al. 2008). La demande WO 2006/107078 décrits différents marqueurs identifiés comme prédictifs d'une réponse à un traitement par interféron chez des patients atteints du VHC.

Il existe toutefois un besoin de pouvoir disposer d'un marqueur prédictif fiable de réponse au traitement à base d'interféron, qui est un traitement contraignant et couteux. Un tel marqueur, dit « théragnostique », serait très utile et permettrait d'ajouter un élément important dans le choix thérapeutique offert aux patients infectés par le virus de l'hépatite C.

### Description des figures

Les figures 1 et 2 représentent la distribution du niveau d'apo-C3, et respectivement, du niveau d'ASH2, chez des patients répondeurs et non-répondeurs à un traitement à base d'interféron.
La figure 3 représente l'aire sous la courbe d'ASH2 et/ou d'apo-C3 (pour les patients répondeurs).

### Description de l'invention

Les inventeurs ont découvert, et c'est le fondement de l'invention, qu'il y a une corrélation entre la présence de l'apolipoprotéine CIII (ci-après, apo-C3) et de celle du dimère de l'albumine sérique humaine (ci-après, ASH2) et la réponse ou non-réponse à une thérapie à base d'interféron chez des patients infectés par le virus de l'hépatite C. Ainsi, selon un premier aspect, l'invention concerne un procédé *in vitro* pour prédire la réponse à un traitement à base d'interféron chez un patient infecté par le VHC, ledit procédé comprenant une étape de mesure du niveau d'apo-C3 dans un échantillon biologique dudit patient préalablement à tout traitement. Typiquement, l'échantillon biologique est un échantillon de sérum ou de plasma.

La réponse au traitement à base d'interféron peut prendre différentes formes : (i) une amélioration de la situation clinique pendant et après le traitement, (ii) une amélioration de la situation clinique pendant le traitement mais une rechute après le traitement, ou (iii) aucune amélioration de la situation clinique pendant ou après le traitement. Les patients visés au (i) seront qualifiés par la suite de patients « répondeurs » (R) alors que les patients visés aux (ii) et (iii) seront qualifiés par la suite de patients « non répondeurs » (NR). L'amélioration de la situation clinique d'un patient se traduit par une diminution (statistiquement) significative de sa charge virale, qui est mesurée par des techniques connues en soi.

Par « interféron » on entend au sens de la présente invention l'interféron-alpha (alpha 2a ou 2b) et ses formes pégylées ou non pégylées, l'interféron-bêta (bêta 1a ou 1b) ainsi que l'interféron-gamma. Selon un mode de réalisation, le traitement à base d'interféron est un traitement à base d'interféron-alpha pégylé ou non pégylé, éventuellement en association avec la ribavirine. Selon un autre mode de réalisation, le traitement à base d'interféron est un traitement à base d'interféron-bêta.

Selon un mode de réalisation, le procédé conforme à l'invention comprend en outre la comparaison du niveau d'apo-C3 mesuré à un niveau seuil d'apo-C3, un niveau d'apo-C3 mesuré inférieur ou égal au niveau seuil étant prédictif d'une réponse au traitement à base d'interféron.

L'apo-C3 est un polypeptide de 8,8 kDa composé d'une séquence mature de 79 acides aminés précédée d'un peptide signal de 20 acides aminés. La digestion enzymatique par la thrombine lors de la coagulation qui est déclenchée lors de la préparation du sérum (mais pas du plasma) clive la séquence mature pour générer un fragment, correspondant aux acides 41-79 (COOH terminal) (Catapano *et al.* 1987). Ce fragment est détecté dans les prélèvements de sérum, et son dosage correspond donc dans ce cas au niveau d'apo-C3 sanguin.

Le niveau seuil d'apo-C3 est établi à partir d'une population de patients infectés par le VHC, chez qui on a prélevé et analysé un échantillon biologique avant le début du traitement à base d'interféron et qui ont été ensuite classés comme patients répondeurs ou non répondeurs, typiquement par détection de leur charge virale au bilan de la 12^{ème} semaine, comme indiqué ci-dessus. Le niveau seuil est la valeur qui permet au mieux de répartir statistiquement la population étudiée entre le groupe R et le groupe NR en utilisant une technologie donnée ; il est ensuite possible, pour déterminer l'appartenance au groupe R ou NR de tout nouveau patient, de mesurer son niveau d'apo-C3 en utilisant la même technologie et de comparer ce niveau au niveau seuil. Bien entendu, le niveau seuil d'apo-C3 est susceptible de varier selon la technique et l'appareillage utilisés pour mesurer les niveaux d'apo-C3, et l'homme du métier sera à même de déterminer ce niveau seuil en fonction du matériel dont il dispose.

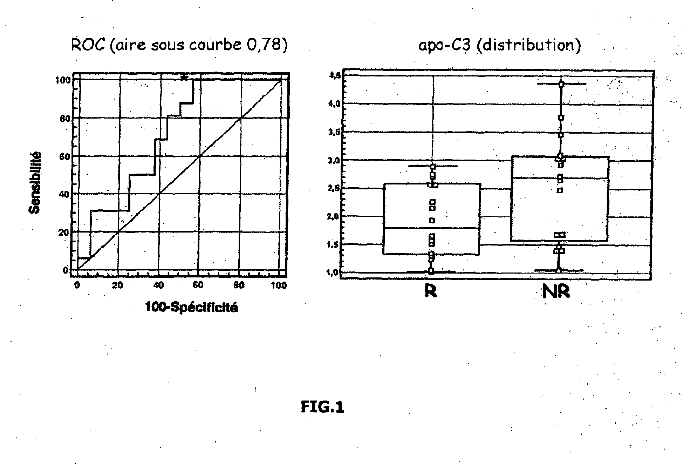

Comme on peut le constater à la lecture de la Figure 1, il existe un seuil statistiquement significatif d'apo-C3 en deçà duquel on peut prédire avec une bonne certitude (haute spécificité) que les patients infectés par le VHC vont répondre à un traitement à base d'interféron.

Selon un autre mode de réalisation, qui peut être combiné avec le mode de réalisation précédent, le procédé conforme à l'invention comprend en outre la comparaison du niveau d'ASH2 mesuré à un niveau seuil d'ASH2, un niveau d'ASH2 mesuré égal ou supérieur au niveau seuil étant prédictif d'une réponse au traitement à base d'interféron.

Le niveau seuil d'ASH2 est établi à partir d'une population de patients infectés par le VHC, chez qui on a prélevé et analysé un échantillon biologique avant le début du traitement à base d'interféron et qui ont été ensuite classés comme patients répondeurs ou non répondeurs, typiquement par détection de leur charge virale au bilan de la 12^{ème} semaine, comme indiqué ci-dessus. Le niveau seuil est la valeur qui permet au mieux de répartir statistiquement la population étudiée entre le groupe R et le groupe NR en utilisant une technologie donnée ; il est ensuite possible, pour déterminer l'appartenance au groupe R ou NR de tout nouveau patient, de mesurer son niveau d'ASH2 en utilisant la même technologie et de comparer ce niveau au niveau seuil. Bien entendu, le niveau seuil d'ASH2 est susceptible de varier selon la technique et l'appareillage utilisés pour mesurer les niveaux d'ASH2, et l'homme du métier sera à même de déterminer ce niveau seuil en fonction du matériel dont il dispose.

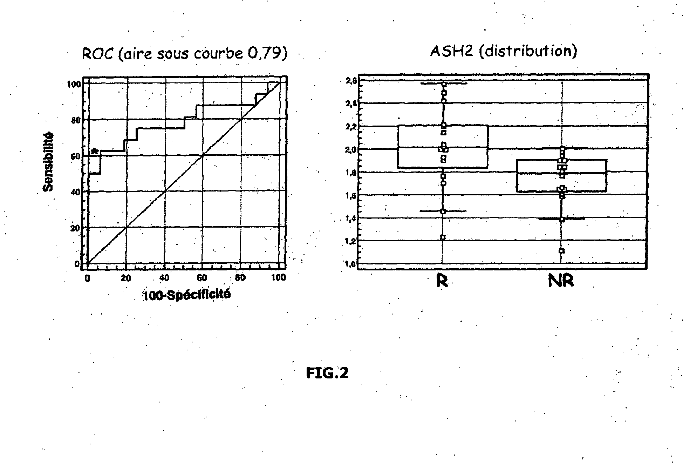

Comme on peut le constater à la lecture de la Figure 2, il existe un seuil statistiquement significatif d'ASH2 au-delà duquel on peut prédire avec une excellente spécificité (93,8%) que les patients infectés par le VHC vont répondre à un traitement à base d'interféron.

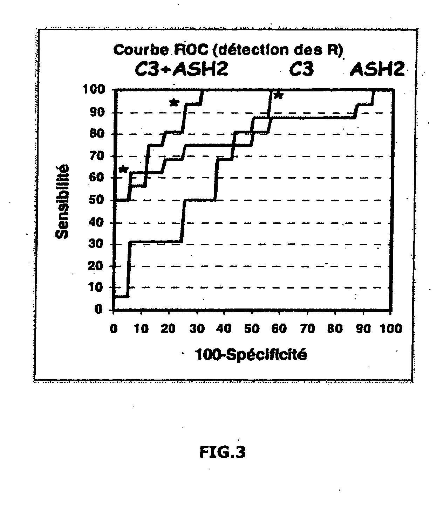

Il est possible d'améliorer la sensibilité du procédé de l'invention en mesurant conjointement les niveaux d'ASH2 et d'apo-C3. Comme on peut le constater à la lecture de la Figure 3, une telle mesure conjointe permet de détecter 93,8% de répondeurs avec une bonne spécificité (75%).

Les niveaux d'apo-C3 et d'ASH2 peuvent être mesurés par une technique de spectrométrie de masse appelée SELDI-TOF (de l'anglais « surface enhanced laser desorption/ionization time-of-flight mass spectrometry »). Cette technique permet de retenir des protéines sur différentes surfaces chromatographiques et de les détecter en spectrométrie de masse. On peut ainsi obtenir rapidement un profil d'expression protéique qui va être analysé grâce à des moyens bioinformatiques. Les pics intéressants sont sélectionnés et les protéines correspondantes identifiées après purification biochimique selon des techniques bien connues de l'homme du métier. On peut ensuite quantifier le niveau des protéines identifiées selon des techniques également bien connues de l'homme du métier (telles que immuno-détection ou spectrométrie de masse quantitative).

Selon un autre mode de réalisation, le procédé conforme à l'invention comprend également la mesure de l'expression ou du niveau d'expression d'au moins un des marqueurs suivants :
- le marqueur génétique IL28B, en particulier le polymorphisme rs12979860 CC et/ou le polymorphisme rs8099917 TT dudit marqueur ;
- les gènes listés dans le tableau 4 de la demande de brevet US 2005/028279, en particulier ceux choisis dans le groupe comprenant ADAR, IFI27, IFI44, OAS3, MX1, MX2, PRKR, IFIT4, TRIM22, G1P2 ;
- HCV-1 ou HCV-4 (US 2010/0158866) ;
- les polynucléotides de séquence n°1, n°2, n°3 ou n°4 de la demande WO 01/71007 ;
- la protéine IP-10 (WO 2008/032210), également connue sous l'appellation CXCL10 ;
- les gènes choisis dans le groupe comprenant KIR3DL3, KIR3DL2, KIR3DL1, KIR2DL1, KIR2DL2, KIR2DL3, KLRG1, KIR3DS1, CD160, HLA-A, HLA-B, HLA-C, HLA-F, HLA-G, IFI27, TNFRSF17, IFI6, OAS2, ISG15, OAS3, IFIT1 (demande de brevet WO 2010/076788).

De manière avantageuse, le procédé conforme à l'invention comprend, outre la mesure du niveau d'apo-C3 et éventuellement d'ASH2, la détection des polymorphismes d'IL28B et/ou la mesure du niveau d'expression de CXCL10.

Le procédé conforme à l'invention permet donc de prédire la réponse à un traitement à base d'interféron chez un patient infecté par le VHC à partir d'un échantillon biologique, typiquement un échantillon de sérum ou de plasma, prélevé chez le patient avant l'instauration du traitement. On comprend donc que le diagnostic de l'infection par VHC a été posé, au moyen de tests classiques (par exemple en mesurant la charge virale, déterminée par les niveaux d'ARN, et/ou le niveau d'antigène capsidique), et que le procédé conforme à l'invention à pour but de déterminer si un traitement à base d'interféron est approprié, c'est-à-dire si un patient est susceptible de tirer ou non un bénéfice d'un tel traitement.

Grâce au procédé conforme à l'invention, on peut proposer à un patient infecté par le VHC, et en particulier par le génotype 1 du virus, un traitement alternatif au traitement à base d'interféron dès lors qu'on a déterminé (par les moyens simples et fiables décrits précédemment) qu'il ne répondra pas audit traitement à base d'interféron. Un tel traitement alternatif peut comprendre l'administration d'au moins un principe actif utile dans le traitement du VHC, choisi notamment parmi les inhibiteurs de protéase, les inhibiteurs de polymérase, les inhibiteurs de NS5A.

Par ailleurs, le fait qu'un patient soit susceptible de répondre ou non à un traitement à base d'interféron va conditionner, à moyen et long terme, l'évolution de sa maladie, ce qui permettra le cas échéant une meilleure organisation de la prise en charge par les équipes médicales compétentes.

Le procédé de l'invention peut être mis en oeuvre à l'aide d'une trousse comprenant :
- des standards permettant l'établissement d'une courbe de référence ;
- un ou plusieurs réactifs spécifiques (anticorps, tampons, réactifs de révélation) nécessaires à la réalisation du dosage quantitatif d'apo-C3 ; et éventuellement
- des instructions pour mettre en oeuvre le dosage quantitatif d'apo-C3 et/ou d'ASH2.

Cette trousse peut comprendre en outre un ou plusieurs réactifs spécifiques (anticorps, tampons, réactifs de révélation) nécessaires à la réalisation du dosage quantitatif d'ASH2. La trousse peut comprendre en outre un réactif spécifique nécessaire au dosage de CXCL10.

L'invention est illustrée par les exemples suivants, donnés à titre indicatif.

### Exemple 1 : mise en évidence de la présence du dimère de l'albumine sérique humaine et de l'apolipoprotéine CIII chez les patients infectés par le VHC

Dans le cadre d'un protocole de recherche clinique du Centre Hospitalier Régional Universitaire de Montpellier validé éthiquement (autorisation du Comité de Protection des Personnes pour la Recherche Biomédicale - CPPRB), des prélèvements ont été effectués chez des patients infectés par le VHC (16 de génotype 1, 3 de génotype 2, 8 de génotype 3 et de 1 de génotype 4, les autres génotypes étant inconnus) préalablement à l'instauration d'un traitement à base d'interféron. Les prélèvements ont été analysés par spectrométrie de masse SELDI.

Pour mettre en évidences les pics d'intérêt, les différents échantillons ont été déposés sur barrette CM10 et Q10 pH7. Le protocole était le suivant :
a) préparation des échantillons : on a mis 10µl de sérum dans 15µl d'une solution urée CHAPS et on a agité 15min pour dénaturer les protéines. On a dilué 7,5µl du mélange dans 300µl de tampon (Tris 100mM pH7 + 0,1% Triton) ;
b) incubation des barrettes : on a placé les 2 barrettes (CM10 et Q10) dans un bioprocesseur. On a ajouté 150µl de tampon (Tris pH7 + 0,1% Triton) sur les spots.

On a incubé 5min sous agitation (500tr/min) puis on a éliminé le tampon et essuyé le bioprocesseur sur papier absorbant.
c) dépôt des échantillons : on a déposé les échantillons dilués sur les spots (8 échantillons par barrette)
d) lavage : on a lavé les spots deux fois 5min avec 150µl de tampon Tris pH7 + 0,1% triton pour enlever les protéines non fixées sur la surface biochimique, puis une fois 5min avec 150µl de tampon Tris pH7 sans triton afin d'ôter le détergent. On a ensuite rincé rapidement les barrettes dans 10ml d'HEPES 5mM pH7 pour éliminer tous les sels et on les a laissé sécher 10min.
e) analyse : on a ajouté 2 x 0,8µl de matrice SPA (et on a laissé sécher 10min entre les 2 applications). L'analyse par SELDI-TOF a été faite à l'aide du spectromètre de masse Protein Chip SELDI System PCS 4000 de la société Biorad. Les barrettes ont été lues en basse masse (3500-20000 Da) et en haute masse (20000-150000 Da) selon deux protocoles de lecture différents :
   - Basse masse (LMW): focalisation sur 10KDa ; énergie du laser 2100 nJ et sur une gamme de masse de 0-200000 Da, avec un tir d'initiation (« warning shot ») à 2300 et ¼ du spot est lu.
   - Haute masse (HMW) : focalisation sur 130kDa, énergie du laser 2300 nJ et sur une gamme de masse de 0-200000 Da, avec un tir d'initiation (« warning shot ») à 2500 et ¼ du spot est lu.

Après avoir passés tous les échantillons au SELDI-TOF, un spectre a été obtenu pour chaque échantillon.

L'analyse a été faite principalement grâce au logiciel Protein chip Data manager. Avant d'analyser les spectres, il faut au préalable :
- aligner les différents spectres ;
- calculer le bruit de fond moyen dans une gamme de masse choisie ;
- ajuster au mieux la ligne de base avant sa soustraction au spectre ;
- normaliser les intensités des spectres en courant d'ion total (TIC).

Une première série d'analyse sur 32 prélèvements a été faite permettant de sélectionner plusieurs pics SELDI d'intérêt (par différence statistique entre groupe R et NR basé sur un test de Student et un test de Mann-Whitney avec p <0,05 au minimum). Une validation utilisant 16 nouveaux prélèvements a permis de confirmer l'intérêt de certains pics (par test de Student et de Mann-Whitney et en calculant la sensibilité et la spécificité obtenue en prenant deux pics en considération pour classer la population) et au final de sélectionner 5 d'entre eux.

Parmi ces 5 pics, l'un d'entre eux nommé p10 a été identifié en spectrométrie de masse après purification biochimique. Un deuxième de ces 5 pics, nommé p4 a également été identifié en spectrométrie de masse après purification biochimique.

La purification des marqueurs p10 et p4 a pu être effectué en reproduisant sur des volumes de sérum plus importants, dans des « spin » colonnes, les conditions de capture du SELDI. Les fractions obtenues ont été analysé en SELDI pour vérifier la présence du marqueur d'intérêt, puis elles ont été chargées sur un gel d'électrophorèse. Après coloration du gel, la zone où le marqueur était attendu du fait de son poids moléculaire (PM) a été excisée. Une dernière vérification de la présence du marqueur élué du gel a été effectuée avant que sa séquence ne soit obtenue par analyse LC-MS/MS.

En ce qui concerne p10, de l'albumine sérique humaine (ASH) a pu être identifiée. Sachant que l'albumine a un PM de 66kDa et que p10 a un PM de 132kDa, on peut conclure que p10 correspond à un dimère d'ASH. p4 quant à lui correspond à une forme tronquée de l'apolipoprotéine CIII. Pour ce dernier marqueur, les peptides séquencés ont été les suivants :
DALSSVQESQVAQQAR (SEQ ID N°:1)
FSEFWDLDPEVRPTSAVAA (SEQ ID N°:2)
GWVTDGFSSLK (SEQ ID N°:3)
DKFSEFWDLDPEVRPTSAVAA (SEQ ID N°:4)

L'alignement de ces peptides sur la séquence de l'apo-C3, précédée de son peptide signal, est illustré ci-dessous :
MQPRVLLVVA LLALLASARA SEAEDASLLS FMQGYMKHAT KTAK**DALSSV QESQVAQQAR GWVTDGFSSL K**DYWSTVK**DK FSEFWDLDPE VRPASAVAA** (SEQ ID N°:5)

La partie séquencée correspond donc au fragment 41-79 de l'apo-C3 que l'on retrouve dans les prélèvements de sérum par suite du clivage à la thrombine lors de la coagulation (Catapano et al. 1987).

### Exemple 2 : corrélation entre présence des marqueurs d'intérêt et réponse ou non réponse au traitement à base d'interféron

Les patients mentionnés à l'exemple 1 ont été traités par interféron-alpha pégylé + ribavirine. Un suivi des patients a été réalisé 12 semaines après le début du traitement, date à laquelle de nouveaux prélèvements ont été effectués afin de déterminer
- d'une part les patients répondeurs (R) et les patients non répondeurs (NR); et
- d'autre part, pour chaque catégorie de patients, s'il existe une corrélation (statistiquement significative) entre les niveaux d'ASH2 et/ou d'apo-C3 déterminés avant l'instauration du traitement et la réponse ou non réponse audit traitement.

Comme on peut le constater à la lecture du tableau 1 et de la Figure 1, il existe une différence statistiquement significative du niveau d'apo-C3 (déterminé avant l'instauration du traitement) entre répondeurs et non répondeurs (p < 0,05 dans le t-test et p < 0,05 dans le test de Kruskal-Wallis). Un niveau d'apo-C3 inférieur au seuil en unité arbitraire de 2,9 permet de détecter 100% de répondeurs avec une spécificité de 43,8% (voir tableau 2), c'est-à-dire qu'aucun non répondeur n'est détecté au-delà de ce seuil.

De même, on peut constater à la lecture du tableau 1 et de la Figure 1 qu'il existe une différence statistiquement significative du niveau d'ASH2 (déterminé avant l'instauration du traitement) entre répondeurs et non répondeurs (p < 0,01 dans le t-test et p < 0,006 dans le test de Kruskal-Wallis). Un niveau d'ASH2 supérieur au seuil de détection en unité arbitraire de 1,9 permet de détecter 62,5% de répondeurs avec une excellente spécificité de 93,8% (voir tableau 2), c'est-à-dire avec très peu de faux-positifs au-delà de ce seuil.

Enfin une combinaison de ces deux marqueurs en prenant comme critère les seuils pour ces deux paramètres permet encore d'améliorer les résultats de détection avec une aire ROC de 0,91 (voir tableau 2 et Figure 3).

**Tableau 1 : Valeurs d'apo-C3 et d'ASH2 mesurées pour les patients R et NR**

| **Groupe** | **Apo-C3** | **ASH2** | **Groupe** | **Apo-C3** | **ASH2** |
|---|---|---|---|---|---|
| R | 1,35 | 1,46 | NR | 2,66 | 1,81 |
| R | 2,57 | 2,15 | NR | 2,72 | 1,67 |
| R | 2,75 | 2,49 | NR | 3,46 | 1,84 |
| R | 2,15 | 2,00 | NR | 3,04 | 1,95 |
| R | 2,90 | 1,71 | NR | 3,77 | 1,90 |
| R | 2,59 | 2,42 | NR | 3,10 | 1,98 |
| R | 1,94 | 2,03 | NR | 3,05 | 2,00 |
| R | 1,65 | 1,77 | NR | 4,36 | 1,61 |
| R | 2,26 | 1,91 | NR | 1,47 | 1,11 |
| R | 1,01 | 1,93 | NR | 1,40 | 1,38 |
| R | 1,52 | 2,21 | NR | 1,69 | 1,76 |
| R | 2,71 | 2,57 | NR | 2,48 | 1,65 |
| R | 1,24 | 1,23 | NR | 2,92 | 1,59 |
| R | 1,30 | 2,20 | NR | 1,41 | 1,85 |
| R | 1,05 | 2,00 | NR | 1,04 | 1,65 |
| R | 1,57 | 2,04 | NR | 1,68 | 1,91 |

**Tableau 2**

| **Pic** | **Sensibilité** | **Spécificité** | **ROC** |
|---|---|---|---|
| Apo-C3 | 100 | 43,8 | 0,72 |
| ASH2 | 62,5 | 93,8 | 0,79 |
| Apo-C3 + ASH2 | 93,8 | 75 | 0,91 |

### Références bibliographiques

Catapano AL. (1987) Activation of lipoprotein lipase by apolipoprotein C-II is modulated by the COOH terminal région of apolipoprotein C-III. Chem Phys Lipids. 1987 Oct;45(1):39-47
Elefsiniotis, I. S., Pavlidis, C., Ketikoglou, I., Koutsounas, S., Scarmeas, N., Pantazis, K. D., Moulakakis, A. & Tsianos, E. V. (2008). Patient's age modifies the impact of the proposed predictors of sustained virological response in chronic hepatitis C patients treated with PEG-interferon plus ribavirin. Eur J Intern Med 19, 266-270.
Hofmann, W. P., Dries, V., Herrmann, E., Gartner, B., Zeuzem, S. & Sarrazin, C. (2005). Comparison of transcription mediated amplification (TMA) and reverse transcription polymerase chain reaction (RT-PCR) for detection of hepatitis C virus RNA in liver tissue. J Clin Virol 32, 289-293.
Ge D, Fellay J, Thompson AJ, Simon JS, Shianna KV, Urban TJ, et al. (2009) Genetic variation in IL28B predicts hepatitis C treatment-induced viral clearance. Nature 2009;461:399-401.
Morgan, T. R., Lambrecht, R. W., Bonkovsky, H. L., Chung, R. T., Naishadham, D., Sterling, R. K., Fontana, R. J., Lee, W. M., Ghany, M. G., Wright, E. C. & O'Brien, T. R. (2008). DNA polymorphisms and response to treatment in patients with chronic hepatitis C: results from the HALT-C trial. J Hepatol 49, 548-556.
Wohnsland, A., Hofmann, W. P. & Sarrazin, C. (2007). Viral déterminants of résistance to treatment in patients with hepatitis C. Clin Microbiol Rev 20, 23-38.
Fujita, N., Nakanishi, M., Mukai, J., Naito, Y., Ichida, T., Kaito, M., Yoshikawa, T. & Takei, Y. (2011). Identification of treatment efficacy-related host factors in chronic hepatitis C by proteinchip serum analysis. Mol Med 17(1-2), 70-78.
Molina, S., Missé, D., Roche, S., Badiou, S., Cristol, JP., Bonfils, C., Dierick, JF., Veas F., Levayer, T., Bonnefont-Rousselot, D., Maurel, P., Coste, J. & Fournier-Wirth, C. (2008). Identification of apolipoprotéine C6III as a potential plasmatic biomarker associated with the resolution of hepatitis C virus infection. Proteomics Clin. Appl. 2, 751-761.

## Revendications

1. Procédé *in vitro* pour prédire la réponse à un traitement à base d'interféron chez un patient infecté par le virus de l'hépatite C (VHC), ledit procédé comprenant une étape de mesure du niveau d'apo-C3 dans un échantillon biologique dudit patient préalablement à tout traitement.

2. Procédé selon la revendication 1, qui comprend en outre une étape de mesure du niveau d'ASH2.

3. Procédé selon la revendication 1 ou la revendication 2, dans lequel on mesure le niveau d'apo-C3 et on compare ce niveau à un niveau seuil d'apo-C3, un niveau d'apo-C3 mesuré inférieur ou égal au niveau seuil étant prédictif d'une réponse au traitement à base d'interféron.

4. Procédé selon l'une des revendications 2 à 3, dans lequel on mesure le niveau d'ASH2 et on compare ce niveau d'ASH2 à un niveau seuil d'ASH2, un niveau d'ASH2 mesuré égal ou supérieur au niveau seuil étant prédictif d'une réponse au traitement à base d'interféron.

5. Procédé selon l'une quelconque des revendications 1 à 4, dans lequel le patient est infecté par le génotype 1 du VHC.

6. Procédé selon l'une quelconque des revendications 1 à 5, dans lequel le traitement à base d'interféron est un traitement par interféron-alpha pégylé ou non pégylé, par interféron-bêta ou par interféron-gamma.

7. Procédé selon la revendication 6, dans lequel le traitement à base d'interféron est un traitement par interféron-alpha pégylé ou non pégylé.

8. Procédé selon la revendication 7, dans lequel l'interféron-alpha pégylé ou non pégylé est utilisé en association avec la ribavirine.

9. Procédé selon l'une des revendications 1 à 8, dans lequel l'échantillon biologique est un échantillon de sérum ou de plasma.

10. Procédé selon l'une des revendications 1 à 9, qui comprend en outre la détection des polymorphismes d'IL28B et/ou la mesure du niveau d'expression de CXCL10.

## Patentansprüche

1. *In vitro*-Verfahren zur Vorhersage des Ansprechens auf eine Behandlung auf Interferon-Basis bei einem mit dem Hepatitis-C-Virus (HCV) infizierten Patienten, wobei das Verfahren einen Schritt zur Messung des Apo-CIII-Niveaus in einer biologischen Probe des Patienten vor jeder Behandlung umfasst.

2. Verfahren nach Anspruch 1, das ferner einen Schritt zur Messung des HSA2-Niveaus umfasst.

3. Verfahren nach Anspruch 1 oder Anspruch 2, wobei das Apo-CIII-Niveau gemessen wird und dieses Niveau mit einem Apo-CIII-Schwellenniveau verglichen wird, wobei ein gemessenes Apo-CIII-Niveau kleiner als das oder gleich dem Schwellenniveau für ein Ansprechen auf die Behandlung auf Interferon-Basis prädiktiv ist.

4. Verfahren nach einem der Ansprüche 2 bis 3, wobei das HSA2-Niveau gemessen wird und dieses HSA2-Niveau mit einem HSA2-Schwellenniveau verglichen wird, wobei ein gemessenes HSA2-Niveau gleich dem oder größer als das Schwellenniveau für ein Ansprechen auf die Behandlung auf Interferon-Basis prädiktiv ist.

5. Verfahren nach einem der Ansprüche 1 bis 4, wobei der Patient mit dem Genotyp 1 des HCV infiziert ist.

6. Verfahren nach einem der Ansprüche 1 bis 5, wobei die Behandlung auf Interferon-Basis eine Behandlung mit pegyliertem oder nicht pegyliertem Interferon-alpha, mit Interferon-beta oder mit Interferon-gamma ist.

7. Verfahren nach Anspruch 6, wobei die Behandlung auf Interferon-Basis eine Behandlung mit pegyliertem oder nicht pegyliertem Interferon-alpha ist.

8. Verfahren nach Anspruch 7, wobei das pegylierte oder nicht pegylierte Interferon-alpha in Kombination mit Ribavirin verwendet wird.

9. Verfahren nach einem der Ansprüche 1 bis 8, wobei die biologische Probe eine Serum- oder Plasmaprobe ist.

10. Verfahren nach einem der Ansprüche 1 bis 9, das ferner die Detektion der IL28B-Polymorphismen und/oder die Messung des Expressionsniveaus von CXCL10 umfasst.

## Claims

1. An *in vitro* method for predicting the response to an interferon-based treatment in a patient infected with hepatitis C virus (HCV), wherein said method comprises a step of measuring the level of apo-C3 in a biological sample from said patient prior to any treatment.

2. The method according to claim 1, which further comprises a step of measuring the level of ASH2.

3. The method of claim 1 or claim 2, in which the level of apo-C3 is measured and compared with a threshold level of apo-C3, wherein a measured level of apo-C3 which is less than or equal to the threshold level is predictive of a response to the interferon-based treatment.

4. The method of claim 2 or claim 3, in which the level of ASH2 is measured and compared with a threshold level of ASH2, wherein a measured level of ASH2 which is greater than or equal to the threshold level is predictive of a response to the interferon-based treatment.

5. The method according to any one of claims 1 to 4, wherein the patient is infected with HCV genotype 1.

6. The method according to any one of claims 1 to 5, wherein the interferon-based treatment is a treatment with pegylated or non-pegylated interferon-alpha, with interferon-beta, or with interferon-gamma.

7. The method of claim 6, wherein the interferon-based treatment is a treatment with pegylated or non-pegylated interferon-alpha.

8. The method of claim 7, wherein the pegylated or non-pegylated interferon-alpha is used in combination with ribavirin.

9. The method according to any one of claims 1 to 8, wherein the biological sample is a serum or plasma sample.

10. The method according to any one of claims 1 to 9, which further comprises detecting IL28B polymorphisms and/or measuring the expression level of CXCL10.
